# EUROPEAN PATENT APPLICATION

(11) **EP 2 266 653 A1**
(43) Date of publication of application: **29.12.2010**
(21) Application number: 09723729.1
(22) Date of filing: 30.03.2009
(51) Int. Cl.: A61M 16/18

(54) **CARTRIDGES OF LIQUID ANAESTHETIC AND VAPORISER**

(30) Priority: 28.03.2008 US 40619 P; 30.03.2009 US 414530
(71) Applicant: ALANDRA MEDICAL, S.A.P.I. DE C.V., México, D.F: (MX)
(72) Inventor: SACRISTAN ROCK, Emilio, México D.F. (MX); HERNÁNDEZ HERNÁNDEZ, Fernando Fabian, México D.F. (MX); RODRIGUEZ SIERRA, Carlos Alfonso, C.P. 15530 México D.F. (MX); SAAVEDRA ROMAN, José Guillermo, C.P. 14100 México D.F. (MX)
(74) Representative: Gemmell, Peter Alan
(86) International application number: PCT/MX2009/000030
(87) International publication number: WO 2009/120057

(57) **Abstract**

A vaporizer and cartridge system are disclosed. The vaporizer may be considered a universal vaporizer in that the vaporizer may use several different liquid anesthetic materials rather than just one. The cartridges may be single-use cartridges that are disposed after use rather than refilled by an end user. The cartridges may have one or more memory devices within the cartridge so that information about the cartridge may be communicated to the vaporizer such as the type of liquid anesthetic material carried by this cartridge. The vaporizer/cartridge system may be implemented to allow the vaporizer to update information in the cartridge memory such as the amount of liquid anesthetic material that was consumed during a particular session of use of the cartridge. The vaporizer may maintain a virtual logbook with information about the operation of the vaporizer. The information in the virtual logbook about the operation of a vaporizer with respect to a particular provision of anesthetic vapor to a particular patient may be communicated to a location outside of the vaporizer for storage along with other related medical records.

## Description

This application claims priority under 35 U.S.C § 119(e) to United States Provisional Application Serial No. 61/040,619, titled "Universal Anesthesia Administration System Consisting of Electronic Vaporizer with Disposable Cartridges," filed on March 28, 2008, which is incorporated into this application by reference in its entirety.

### Field of The Invention

This invention relates generally to the provision of anesthesia. More specifically to precision vaporizers that provide vapors from liquid forms of anesthetic material.

### BACKGROUND OF THE INVENTION

Most of the inhalational anesthetic agents in current use are liquids at atmospheric pressure and room temperature. These liquid anesthetic materials need to be converted into vapor for use in the gas provided to the patient to be anesthetized. It is important that a vaporizer provide vapor in keeping with the specific amount requested by the end user as too much anesthesia can be deleterious to the patient and too little anesthesia may lead to the patient becoming less than fully anesthetized during the procedure.

Additional problems that are possible with vaporizers is that the vaporizer runs out of liquid anesthetic material without warning and the end user is not prepared to quickly remedy the situation which may lead to the patient becoming less than adequately anesthetized.

As many vaporizer systems allow the end user to refill the reservoir that holds the liquid anesthetic material, there is a risk that the potent liquid anesthetic material may be spilled outside of the vaporizer or the reservoir, thus polluting the ambient air in the operating room. There is also a risk that during the process of refilling a reservoir, that liquid anesthetic material may enter the tubing within the vaporizer so that droplets containing large amounts of liquid anesthetic material relative to the precisely metered vapor may get into the gas provided to the patient thus providing a sudden spike in the amount of anesthetic agent provided to the patient.

While vaporizers exist for a number of different liquid anesthetic agents, these vaporizers are agent specific. Thus, a hospital needs to maintain vaporizers for each anesthetic agent that is used in that hospital as there is not a "universal" vaporizer that may be used with many different liquid anesthetic agents.

A common problem with existing vaporizers is the lack of correction for differences in atmospheric pressure. Hospitals operating at significant elevations have differences in atmospheric pressure as compared with hospitals operated near sea level.

### SUMMARY

A vaporizer and cartridge system are disclosed. The vaporizer may be considered a universal vaporizer in that the vaporizer may use several different liquid anesthetic materials rather than just one. The cartridges may be single-use cartridges that are disposed after use rather than refilled by an end user. The cartridges may have one or more memory devices within the cartridge so that information about the cartridge may be communicated to the vaporizer such as the type of liquid anesthetic material carried by this cartridge. The vaporizer/cartridge system may be implemented to allow the vaporizer to update information in the cartridge memory such as the amount of liquid anesthetic material that was consumed during a particular session of use of the cartridge.

The vaporizer may maintain a virtual logbook with information about the operation of the vaporizer. The information about the operation of a vaporizer with respect to a particular provision of anesthetic vapor to a particular patient may be communicated to a location outside of the vaporizer for storage along with other related medical records.

This summary is meant to introduce the concepts that are disclosed within the specification without being an exhaustive list of the many teachings and variations upon those teachings that are provided in the extended discussion within this document. Thus, the contents of this summary should not be read as a limit to the scope of the claims that follow.

Other systems, methods, features, and advantages of the present invention will be or will become apparent to one with skill in the art upon examination of the following figures and detailed description. It is intended that all such additional systems, methods, features and advantages be included within the scope of and be protected by the accompanying claims.

### BRIEF DESCRIPTION OF THE FIGURES

The invention can be better understood with reference to the following figures. The components in the figures are not necessarily to scale, emphasis instead being placed upon illustrating the inventive principles. Moreover, in the figures, like reference numerals designate corresponding parts throughout the different views.

FIG. 1 is a flow chart of a method for using a vaporizer and cartridge.

FIG. 2 is a flow chart of a method of replacing a primary cartridge.

FIG. 3 shows one example of components used in the production of gas carrying anesthetic vapor.

FIG. 4 illustrates the components in communication with the vaporizer control system.

FIG. 5 is a front view of a vaporizer with both a primary and a spare cartridge.

FIG. 6 is a top view of a vaporizer with both a primary and a spare cartridge.

FIG. 7 is a rear view of a vaporizer.

FIG. 8 is a left side view of a vaporizer with both a primary and a spare cartridge.

FIG. 9 is a right side view of a vaporizer with both a primary and a spare cartridge.

FIG. 10 is a bottom view of a vaporizer with both a primary and a spare cartridge.

FIG. 11 is a perspective view of the top, left, and front sides of a vaporizer with both a primary and a spare cartridge.

FIG. 12 is a perspective view of the top, right, and front sides of a vaporizer with both a primary and a spare cartridge.

### DETAILED DESCRIPTION

In the following description of examples of implementations, reference is made to the accompanying drawings that form a part hereof, and which show, by way of illustration, specific implementations of the invention that may be utilized. Other implementations may be utilized and structural changes may be made without departing from the scope of the present invention.

The present invention relates to a method for the generation of vapor from liquid anesthetic material. Gas flow with a controlled amount of anesthesia vapor is provided to equipment known to those skilled in the art which in turns provides the anesthesia to the patient receiving the anesthesia as part of a surgical procedure.

After setting forth a method of using an electronic vaporizer and cartridges loaded with liquid anesthetic material, examples of the electronic vaporizer with inserted cartridges are provided. One of skill in the art will recognize that the examples provided on the specific shape of the preloaded cartridge may be modified in accordance of the teachings of the present invention so that the cartridge looks different from the cartridge that is disclosed while providing some or all of the benefits associated with the present invention.

Method of Use.

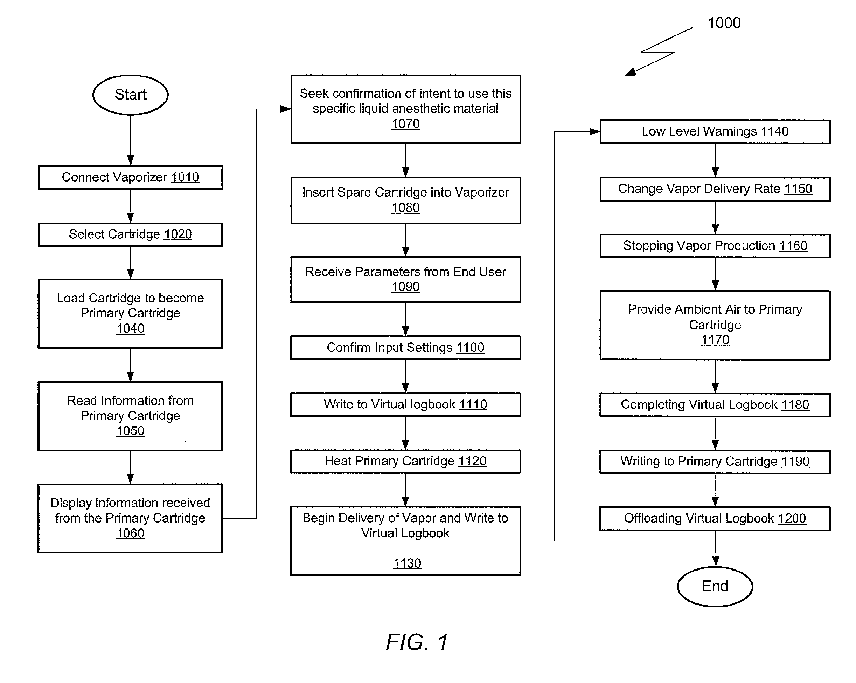

A method of utilizing a cartridge and vaporizer is set out as a process described in FIG. 1.

Step 1010-Connect Vaporizer.

The electronic vaporizer is connected into the system of anesthesia equipment. An anesthesia system can be summarized at a high level as a fresh gas supply, a vaporizer, and a breathing circuit. Many new systems have data interfaces with an Anesthesia Information Management System (AIMS) to collect and store a wide range of information about the anesthesia provided to a particular patient to make that part of the patient's record for the surgery. Many systems have a number of additional safety features and redundancies such as systems that have several vaporizer units and an interlock to ensure that only one vaporizer is providing vapor at any given time. As anesthesia systems are made of a series of components, there are standard mounting systems such a back bars that receive components.

A vaporizer made in accordance with one or more teachings of the present invention may include a capacity to be mounted to a back bar and work with a standard interlock system so that a series of vaporizers may be used in a given anesthesia system while preventing more than one vaporizer from simultaneously providing vapor to the gas stream going to the breathing circuit. By way of example but not limitation, this would include a Selectatec® brand bar and work with Selectatec® compatible interlock systems.

The vaporizer may have an onboard battery to minimize the impact of temporary power interruptions. The vaporizer may have a cord to connect with an electric outlet and a power supply to provide the power needed for operation of the vaporizer and the charging of any rechargeable battery associated with the vaporizer.

Step 1020-Select Cartridge.

A cartridge preloaded with a particular liquid anesthetic material is selected for use in a surgical procedure. The cartridge may have a color used to provide a visual indication to the end user of the liquid anesthetic material contained inside. Some systems may be adapted to use a single liquid anesthetic material. For a given liquid anesthetic material, the liquid has a tendency to release molecules from the liquid into the gas above the liquid until the vapor reaches a saturated vapor pressure which is a function of the liquid and of the temperature. The predictability of saturated vapor pressure (based upon the liquid used and the temperature heated temperature of the liquid, and the ambient air pressure) allows the creation of carrier gas containing anesthesia vapor.

The carrier gas may be mixed with bypass gas (which does not enter the vaporizing chamber) to create an output that is in turn provided to the breathing circuit.

The cartridge may contain any liquid anesthetic material that may be used with a heat controlled vaporizing system. Examples of liquid anesthetic material that may be suitable for use with the present invention include: Halothane, Enflurane, Isoflurane, Desflurane, and Sevoflurane.

Step 1040-Load Cartridge.

The cartridge is loaded into the vaporizer cartridge cavity to become the primary cartridge. The process of loading opens the hermetic seal that has been keeping the volatile liquid anesthetic material from vaporizing and providing anesthesia to the ambient air. The connection between the cartridge and the vaporizer unit may be implemented so as to be simple and reliable so that the connection may be established without any effort by the users in order to engage the cartridge and the connection needs to close off when the cartridge is removed as simply and reliably as a Schrader valve used for many bicycle and automobile tires.

Step 1050-Receive Information from Primary Cartridge.

It is preferred that the electronic vaporizer be able to receive directly from the primary cartridge the relevant information about the contents of the primary cartridge including but not limited to the specific liquid anesthetic material within the primary cartridge. Other information that may be passed from the primary cartridge memory to the vaporizer includes: lot number, expiration date, and the volume of liquid anesthetic material inserted at the factory. Those of skill in the art will recognize that other parameters could be stored on the primary cartridge and used by the vaporizer to provide the same functionality or to provide additional functionality. For example, the creation date of a cartridge could be used by a system that could determine the expiration date based on the creation date and other relevant information.

Optionally, upon insertion into the vaporizer, the vaporizer could communicate to the memory on the primary cartridge that the primary cartridge had been inserted into a vaporizer and this information stored within the primary cartridge and subsequently communicated should the primary cartridge be removed from the vaporizer and subsequently inserted into this or another vaporizer.

The information from the primary cartridge including the amount of liquid anesthetic material placed into the primary cartridge at fabrication (or remaining in the primary cartridge after an earlier use) is stored along with a time stamp as part of a virtual logbook for the vaporizer.

Amount may be expressed as volume such as cubic centimeters, weight, capacity such as milliliters; or any other measurement that is reasonable for describing an amount of liquid anesthetic material.

The information from the primary cartridge could be conveyed to the vaporizer over a conventional wired data bus or conveyed in a wireless manner such as via RFID technology. RFID technology (Radio-Frequency Identification) is used in a wide array of applications for inventory control. Some RFID technologies use active devices which contain their own power source and others use passive RFID devices that interact with another powered device that causes the transfer of data without reliance on power at the passive device.

Wireless communication embraces a wide range of technologies and protocols including Bluetooth short range communications, Wi-Fi, and a range of proprietary protocol stacks such as the SimpliciTI™ network protocol for simple, low power networks provided by Texas Instruments, Inc.

Step 1060-Display Information.

Information from the primary cartridge is displayed for viewing by the end user. The vaporizer may have a built in display such as a liquid crystal display or other suitable display. Examples of information from the primary cartridge that may be displayed upon insertion of the primary cartridge include the liquid anesthetic material present in the primary cartridge. One of ordinary skill in the art will appreciate, that the identity of the liquid anesthetic material may be conveyed using a product code but displayed with a text string or other format that is not an exact copy of the information provided from the primary cartridge.

Step 1070-Confirm Intent to Use Specific Liquid Anesthetic Material.

The end user may be asked to confirm to the vaporizer that the end user intends to use the specific liquid anesthetic material that the primary cartridge reported to the vaporizer is present in the primary cartridge. The type of liquid anesthetic material would be displayed or otherwise conveyed to the end user before the end user can acknowledge intent to use that material.

Step 1080-Receive Spare Cartridge.

Optionally, the vaporizer housing may be adapted to receive a second cartridge so that a spare cartridge is present in a known place rather than loose in the operating room. In a vaporizer with two places for cartridges, the cartridge in position for use by the vaporizer may be called the primary cartridge and the second cartridge may be called the spare cartridge. Other than the current use of the cartridge, the cartridges may be identical.

This spare cartridge would be available to use so that an exhausted or near exhausted primary cartridge may be removed from the active engagement with the vaporizer and the spare cartridge inserted immediately thereafter. Insertion changes the spare cartridge into the new primary cartridge. After the spare cartridge had been placed in service, the end user could request that an additional cartridge be brought from the place where they are stored and inserted into the vaporizer as the new spare cartridge.

Optionally, the vaporizer may be configured to read the information stored on the spare cartridge stored in the vaporizer so that the spare cartridge can be checked to make sure that the spare cartridge has the same liquid anesthetic material as the current primary cartridge and the liquid anesthetic material is not expired. In systems that store the remaining volume on the cartridge as a primary cartridge is removed from use by the vaporizer, the spare cartridge placed in the spare cartridge portion of the vaporizer may be checked to see how much volume is thought to be on the spare cartridge and this information can be displayed.

Step 1090-Receive End User Input.

The end user sets the desired flow rates and vapor concentration. The electronic vaporizer will have a display in order to provide indications of the liquid anesthetic material in the cartridge and other relevant information. The end user will interact with the electronic vaporizer through input mechanisms known in the art. Examples include buttons that allow the user to move up or down through a list and otherwise navigate through and select items in a series of menus. Alternatively, touch screens may be used to both display choices and accept end user input. The electronic vaporizer may include one or more large rotary decoders that allow for the rotation of a knob or dial to input the rate of vaporized liquid anesthetic material.

The primary feedback to the end user on the amount of anesthesia selected for delivery may be handled on a display screen. A common way of expressing this concept is through the percent concentration of vapor in the outlet gas of the vaporizer unit that is then provided to the breathing circuit. The outlet gas is a mix of carrier gas that is exposed to the working portion of the vaporizer and carries anesthesia vapor and bypass gas that does not get exposed to the working portion of the vaporizer and thus does not pick up anesthesia vapor.

The display may indicate: the liquid anesthetic material in use; the selected dose rate; the estimated amount of remaining liquid anesthetic material; the estimated time to exhaust the remaining liquid anesthetic material at the current dose rate; a comparison of the current dose rate against a scale of the allowable range of dose rates for the liquid anesthetic material in use; and indications of the current status of the vaporizer.

The end user may request that the display indicate: the average dose rate over the course of this procedure; a value indicating the amount of time since the procedure was started; an alarm log; and information regarding the maintenance of the vaporizer such as when the vaporizer was last inspected.

The dose may be expressed in percentage of vapor with respect to the outlet gas of the vaporizer which is a mix of the carrier gas carrying vapor and the bypass gas.

As the percentages of vapor used for one anesthetic material may in the range of zero to five percent but for another anesthetic material, the safe range may be zero to twenty percent, it may be best to rely exclusively on the data provided in the display rather than try to come up with a scale on a rotary knob as it may be difficult to use one scale for an array of different liquid anesthetic materials.

Step 1100—Confirm Input Settings.

The end user reviews and confirms a range of parameters received by the vaporizer to ensure that the vaporizer is being asked to prepare to deliver what is needed for the patient.

Step 1110-Write to Virtual Logbook.

These confirmed parameters are also made available to the virtual logbook. The parameters may include: the liquid anesthetic material in use; the selected dose; scale of permissible concentrations for that particular liquid anesthetic material; amount of material remaining (in whatever unit or form is useful to the end user); current status of the vaporizer and other parameters. Examples of other parameters that may be displayed include: average rate of use of the liquid anesthetic material; remaining time for use of this cartridge at the current rate of use; timer showing the running time on the procedure; and alarm status. Although not normally needed during a procedure, the vaporizer may be queried and display the last time the vaporizer was serviced and the amount of time until the next required servicing (in calendar days or working hours depending on how the maintenance routine is calculated).

The alarms calculated and displayed by the vaporizer may include conventional alarms found in prior art vaporizers. The alarms may provide a warning that the cartridge is within a few minutes of exhaustion. The alarms may note interruption of power such that the vaporizer is operating on battery power. The alarms may note a change in the condition of the input gas such as a significant drop in pressure.

One of skill in the art will recognize that certain clusters of information are likely to be displayed as the default display during a surgical procedure and that other combinations of information may be selected for temporary or ongoing display based upon user input.

After receiving the input from the end user on the initial desired percent concentration of the anesthetic material in the outlet gas, there may be a short delay as the cartridge is heated to provide the desired vapor supply. After the cartridge is adequately heated, one or more control valves open to direct the carrier and bypass gas to provide a supply to the breathing circuit. The end user inputs are made available to the virtual logbook.

Step 1120-Heat Primary Cartridge.

The cartridge is heated to the required temperature to provide the rate of vapor necessary to meet the request of the end user. The heating of the cartridge content may be primarily from having a thermally conductive material surrounding the liquid anesthetic material (such as a metal) in the interior of the cartridge that is connected by a thermally conductive material to the exterior of the cartridge so that a path for conductive heat transfer is established.

While it is a design objective to provide one or more paths for heat to travel from the vaporizer into the primary cartridge, it is another design objective to minimize the risk that the end user may touch hot portions of the primary cartridge while removing an exhausted or nearly exhausted primary cartridge after use. The cartridge may have electric strip heat built into the external walls of the reservoir holding the liquid anesthetic material so that a variable amount of electricity may be provided to the strip heat to warm the reservoir and the reservoir contents. A cartridge may use a combination of a conductive path for heat to flow from the vaporizer into the cartridge combined with strip heat to warm the interior of the cartridge.

After receiving the input from the end user on the initial desired percent concentration of the anesthetic material in the outlet gas, there may be a short delay as the cartridge is heated to provide the desired vapor supply. After the cartridge is adequately heated, one or more precision control valves open to direct the carrier and bypass gas to provide a supply to the breathing circuit.

The control system for providing heat to the primary cartridge may rely on a temperature reading, but other implementations may be configured to make primary reliance on a pressure reading as the pressure will indicate whether the primary cartridge is receiving sufficient heat to provide adequate vapor (assuming that the primary cartridge is not exhausted).

The vapor pressure may be measured when the valves to the cartridge are open and the valves to the vaporizer are closed.

One or more precision valves on the output of the vaporizer can be controlled by the vaporizer microcontroller so that the valves open only if the various parameters and checks indicate that it is appropriate to open the valves.

Step 1130-Start of Vapor Delivery.

The start of vapor delivery is noted to the virtual logbook including parameters regarding the creation of the vapor such as operating pressure and any measured temperatures. Once the first entry is provided to the virtual logbook, a system may call for providing periodic snapshot readings of all important parameters at set time intervals and augmented by readings taken on specific events such as an alarm or a removal of the cartridge or the receipt of an instruction to change the output rate including stopping the production of vapor.

Step 1140-Low level warnings.

In the event that the pressure measurement drops below the requested pressure for the rate of vapor desired and the temperature measurements indicate that adequate heat is being provided to generate the requested rate of vapor then the system will interpret this fact pattern as indicating an exhausted or nearly exhausted primary cartridge and provide a prescribed alarm. The alarm can be provided using any type of alarm known to those of skill in the art including using color, flashing, text, and possibly audible alarms to get the attention of the end user. Most systems will note to the virtual logbook the time and details of the low level warning.

In many implementations, the system will be calculating the remaining liquid anesthetic material given what the system was provided about the initial volume in the primary cartridge and the aggregate use during the procedure so that the end user is receiving an estimated time to primary cartridge exhaustion so that a pressure drop from an exhausted cartridge is not needed in order to prompt the end user to replace the primary cartridge. Alarm warnings may be provided to the end user when the system calculates a prescribed number of minutes of vapor provision from this primary cartridge. The provision of this alarm warning may be noted to the virtual logbook. Additional alarms may be provided and noted to the virtual logbook. For example a warning may be provided at ten minutes until exhaustion with alarms provided at five minutes until exhaustion and again at one minute until exhaustion.

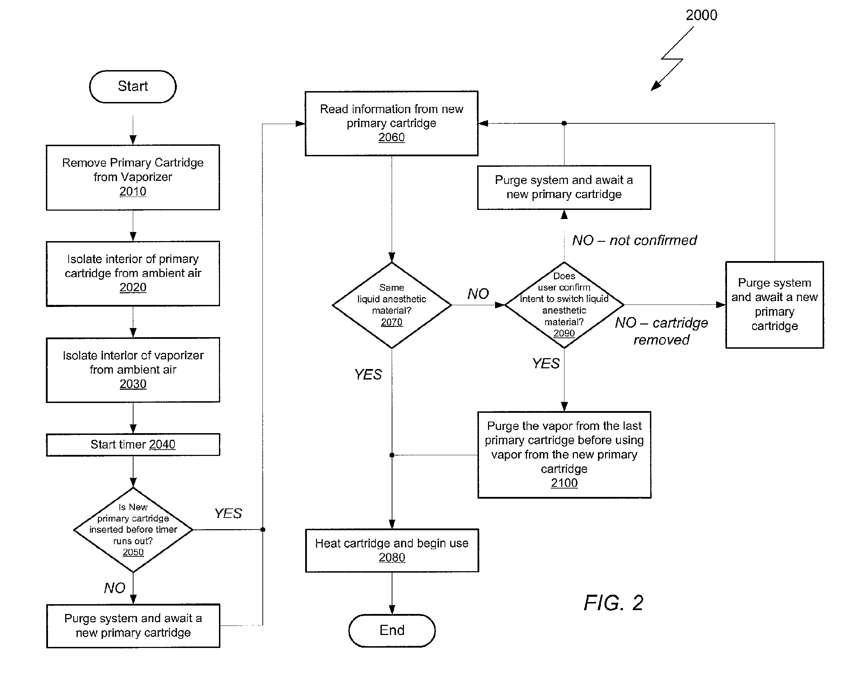

Note that even a system that has calculated times to exhaustion may monitor for a pressure drop indicative of exhaustion as it is possible that for some reason the primary cartridge did not receive at the factory the prescribed amount of liquid anesthetic material so that the cartridge may become exhausted earlier than predicted. The process for replacing a primary cartridge with a new primary cartridge is addressed below and in FIG. 2.

Step 1150-Changing the vapor delivery rate during a procedure.

The vaporizer may be implemented so that the precise control of the release of vapor is primarily controlled by the opening and closing of control valves to release vapor from the cartridge side of the control valve into the carrier gas. As the pressure difference is known across the valve and the flow characteristics through the valve are known, the amount of vapor released to the carrier gas with each short opening of the control valve is readily calculated. In response to a request from the end user to change the rate of vapor provided, the control valve will be open a greater percentage of time (through some combination of more frequent openings or slightly longer duration openings). Temperature set point for the primary cartridge will be adjusted upward if needed in order to increase the rate of vaporization if needed to maintain the desired vapor pressure.

Step 1160-Stopping the Vaporizer. Upon a request from the end user to set the vapor rate to zero, the control valves close and stop opening so that no more vapor is received from the cartridge. No more heat is provided to the primary cartridge. A purge cycle is started to route fresh gas through the interior of the vaporizer before the vaporizer cools off sufficiently to have the residual liquid anesthetic material condense in the vaporizer. A final set of data is sent to the virtual logbook.

Step 1170-Providing Ambient Air to the Primary Cartridge

As the primary cartridge cools, the pressure within the primary cartridge from the vapor pressure of the liquid anesthetic material will drop as some of the vapor returns to liquid form. In order to avoid having a significant vacuum within the cooling cartridge, a pressure release valve allows ambient air into the cartridge. The pressure release valve may be in series with one or more one way valves such as a check valve to provide additional protection against the leakage of anesthetic vapor into the ambient air.

Step 1180-Completing the Virtual Logbook Entries for the Procedure.

A request to stop the provision of vapor may trigger a snapshot of the status of the vaporizer as the vaporizer stops providing vapor. Optionally, a set of summary data may be sent to the virtual logbook to summarize the operation of the vaporizer for this procedure. The various pieces of information provided to the virtual logbook will allow for the interpretation of the logbook to discern the length of time that anesthesia was provided by this vaporizer, the total amount of anesthesia, and the provision rates of vapor over time during the procedure. The virtual logbook may contain indications of the times and types of alarms along with the responses to the alarms by the end user including the type of response and the delay between the provision of the alarm and the receipt of the response.

Step 1190—Writing Data to the Primary Cartridge.

Optionally, information about the use of the primary cartridge in use at the end of the procedure may be written to memory on the primary cartridge. The information written to the primary cartridge may be the estimated amount of liquid anesthetic material left in the cartridge based upon the quantity of liquid anesthetic material reported to the vaporizer minus the calculated consumption of the liquid anesthetic material during this procedure.

A system may be established that calls for certain memory locations to indicate that the cartridge is in use. These memory locations would be updated by the vaporizer at the end of use of a cartridge and before removal. If a primary cartridge is removed before a final update to the memory of the primary cartridge, then the cartridge memory may still indicate that the cartridge is in use. The next time that this cartridge is inserted for use by a vaporizer, the vaporizer may recognize that this cartridge was removed prematurely before a final memory update and thus information on the cartridge memory may be incomplete. The vaporizer may be programmed to warn the end user to remove the cartridge and use one without memory problems.

The vaporizer system and related procedures may be implemented to not reuse a cartridge so that each procedure uses only cartridges that have not been used as primary cartridges and actually provided vapor in any previous procedure. If the procedure was to not reuse a cartridge after any liquid anesthetic material had been provided to the vaporizer, there would be no need to write anything to the primary cartridge beyond an indication that it had been used to provide vapor. The notation written to the primary cartridge that it had been used could be written as soon as the primary cartridge was inserted in the primary cartridge portion of the vaporizer or when the control valves open to allow out vapor from the primary cartridge. In some implementations, the vaporizer may write to the cartridge as soon as the cartridge is inserted into the vaporizer or is used to produce vapor so that the cartridge is marked as not new. If the information is written as soon as the cartridge is not new, then there may not be a need to write not new to the cartridge when the cartridge is about to be removed or just recently removed but within range of the wireless communication system.

Step 1200-Offloading the Virtual Logbook.

The virtual logbook for this surgical procedure is part of the relevant records for this surgical procedure through any of the many conventional methods for movement of data. The data may be written to a memory storage device associated with the vaporizer. The memory device may be a USB (Universal Serial Bus) flash drive. The virtual logbook may be provided via a communication link to an Anesthesia Information Management System (AIMS) to collect and store a wide range of information about the anesthesia provided to a particular patient to make that part of the patient's record for the surgery or to some other medical records management system. After the virtual logbook is successfully communicated either to a memory storage device or to memory associated with the communication link, the virtual logbook for this procedure may be erased automatically from the vaporizer or it may be retained within the vaporizer until manually requested to be erased.

Some information necessary for maintenance records of the vaporizer may be kept in separate memory. The items useful for the maintenance of a medical device are known to those of skill in the art but for sake of illustration may include information such as the hours of use of the device, any alarms received indicative of a problem with the vaporizer rather than merely an exhausted or nearly exhausted primary cartridge, and other information that would be useful in determining when the vaporizer should be serviced and what sort of problems the vaporizer may have experienced since the last service.

PROCESS OF REPLACING THE PRIMARY CARTRIDGE

FIG. 2 contains a sequence of steps in process 2000 for replacing the primary cartridge with another cartridge.

Step 2010-Remove Primary Cartridge.

After receiving an indication that the primary cartridge is exhausted or nearing exhaustion so that it is time to swap out the primary cartridge, the primary cartridge is removed from the vaporizer.

Step 2020-Isolate Reservoir of Removed Cartridge from Ambient Air.

As the end user physically removes the primary cartridge from the vaporizer, the end user needs to be isolated from the anesthetic material remaining in the cartridge. This isolation may happen automatically as it is the physical interaction with the vaporizer that opens a channel to the liquid anesthetic material and removing the cartridge closes this channel, such as through the use of a Schrader valve.

Step 2030-Isolate Vapor in Vaporizer from Ambient Air.

In addition to isolating any remaining liquid anesthetic material from the end user, the system should isolate any vaporized anesthetic material within the vaporizer from the ambient air. The vaporizer may have one or more one way valves to limit the flow of material to passing only from the vaporizer cartridge to the vaporizer interior and not from the vaporizer interior towards the primary cartridge (or the void where a primary cartridge could be placed).

As the control system notes the removal of the primary cartridge from the vaporizer, a control valve between the vaporizer and the cartridge closes. This closed control valve is in addition to the one or more one way valves.

Step 2040-Removal of the Primary Cartridge Starts a Timer.

If a new primary cartridge is not inserted within a prescribed period, an auto purge is triggered to run purge gas through the vaporizer. The purge cycle would prevent condensation of anesthetic vapor within the vaporizer should the vaporizer not receive a new cartridge for an extended period. This timer may be implemented in any conventional way using hardware or software. Depending on the implementation chosen, there may not be a distinct device that is the timer.

Branch 2050.

If a new primary cartridge is inserted before the timer triggers a purge cycle, then continue to Step 2060, else purge the system and await a new primary cartridge.

The vaporizer may be configured to look at the total time between the removal of one cartridge and insertion of the next one. If the total time exceeds a certain prescribed period, then the system may seek from the end user either a confirmation that this new primary cartridge is being used in a continuation of the existing procedure (so it goes into the open virtual logbook) or it is the start of a new procedure so that the end user must enter all the required information to start a new procedure and open a new virtual logbook.

Step 2060-Read information from the new Primary Cartridge.

This new primary cartridge may have been located within a cavity in the vaporizer as a spare cartridge or this new cartridge may have been stored somewhere else. Note that while the spare cartridge may have had its memory read and been checked for a match of liquid anesthetic material and expiration date, the vaporizer checks this information again as it is not assured that the new primary cartridge is the same as the spare cartridge as the cartridge is for a short period of time not in direct contact with the vaporizer and thus could be inadvertently switched with some other cartridge.

Branch 2070-Same Liquid Anesthetic material?

After reading the contents of the cartridge in position to be the new primary cartridge, the vaporizer can determine if this is a continued use of the same liquid anesthetic material. If it is, then the process continues to Step 2080.

Conversely, if the new primary cartridge does not have the same liquid anesthetic material as the immediate past prior material, the control system does not open a control valve between the primary cartridge and the interior of the vaporizer and the vaporizer does not apply any heat to the new primary cartridge and instead asks for confirmation of the change in liquid anesthetic material. If there has been a change of liquid anesthetic material, then the end user is asked to confirm this unusual switch at Branch 2090.

Step 2080-Heat Cartridge and Begin Use.

If the new primary cartridge contains the same type of liquid anesthetic material as was used in the immediately prior primary cartridge and the liquid anesthetic material is deemed appropriate for use (such as not beyond expiration date or not too far from the stored creation date) then the new primary cartridge is exposed to heat to start the vaporizing process at the intended rate. When the control system indicates that the new primary cartridge is ready, the vaporizer may start using vapor from the new primary cartridge and mixing this vapor with the fresh gas and providing the mixture to the breathing circuit.

Step 2080 marks the normal end of the process of changing cartridges.

Step 2090-Branch On Confirmed Intent to Change of Liquid Anesthetic Material.

The end user is alerted to the change in anesthetic material and asked to confirm this choice. If Yes, then go to step 2100. If no, then purge the system and await a new primary cartridge.

In most instances, the switch from one liquid anesthetic material to a second one during a single medical procedure will not be the intended choice. When asked to confirm the switch, the end user will simply remove the incorrect cartridge and insert a different cartridge to become the new primary cartridge to provide more of the liquid anesthetic material of the same type as provided by the immediate prior primary cartridge. Removal of a cartridge instead of confirming intent to use the new cartridge despite the change in material is treated as a no answer. The vaporizer may be set up to force a purge cycle if the incorrect (second) cartridge is removed without providing vapor while the third cartridge is obtained and inserted as there was a delay in getting the correct cartridge in and there is a risk that the vaporizer will cool and allow vapor to condense.

Condensed droplets may make metering of the dose rate inaccurate since a relatively small droplet has the same amount of liquid anesthesia as a relatively large amount of vapor of that same liquid anesthetic material.

Step 2100 Purge Cycle.

If the user answers yes at Branch 2090, then a purge cycle is started. During the purge cycle, a control valve is opened to provide fresh gas near the connection with the vaporizer cartridge. Control valves are opened as needed for the fresh gas to run through the interior of the vaporizer and out the purge exit. After the purge gas leaves the vaporizer, the purge gas may be handled in manners known to those of skill in the art to remove any residual vapor. Purge cycles prevent condensation of vapor of the liquid anesthetic material in the interior of the vaporizer to minimize mixing of two different liquid anesthetic materials or the uncontrolled delivery of droplets of liquid anesthetic material.

After the prescribed purge, the end user may proceed with use of the new primary cartridge with the different liquid anesthetic material. It would be up to the end user to make any required adjustments to the breathing circuit to purge the vapors from the first liquid anesthetic material or to replace the breathing circuit with a different breathing circuit.

SAMPLE PIPING AND CONTROL LAYOUT

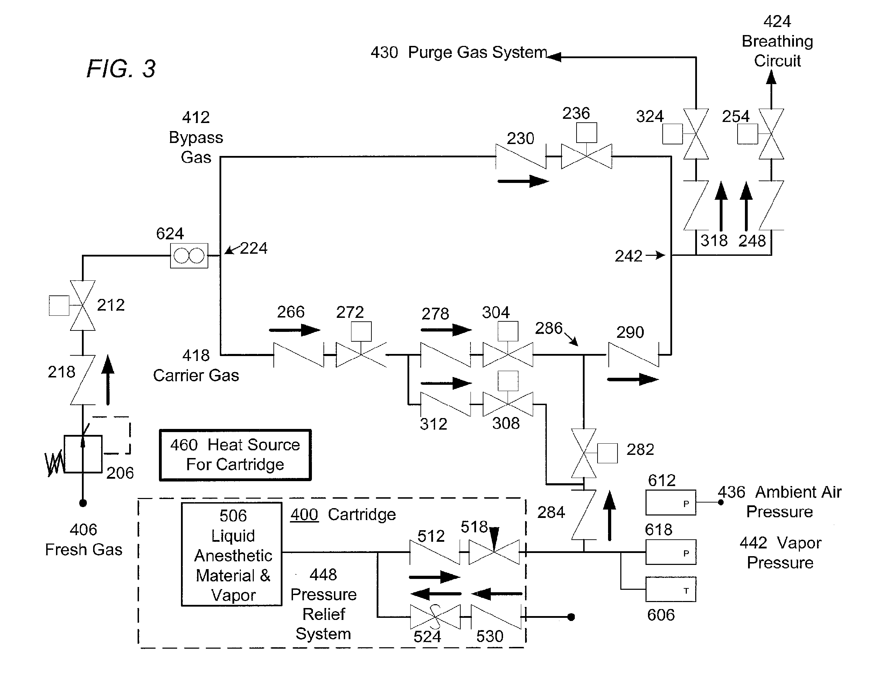

The present invention may be implemented in a variety of ways by one of skill in the art but in order to effectively convey the concepts of the present invention, a sample layout is provided in FIG. 3.

Primary Gas Flows During Vaporization.

A pressure regulator 206 regulates the pressure of the fresh gas 406 provided to control valve 212 connected to one-way valve 218. Fresh gas is limited to traveling towards the vaporizer loop. At junction 224, some gas becomes bypass gas 412 and some becomes carrier gas 418. Carrier gas 418 carries vapor picked up from the vaporizer section and the bypass 412 gas does not go through the vaporizer section. The ratio of bypass gas 412 to carrier gas 418 may be controlled by the relative positions of control valve 236 on the bypass gas path and control valve 272 on the carrier gas path.

The total fresh gas 406 flow (both carrier gas 418 and bypass gas 412) may be measured at flowmeter 624 located just before junction 224. One of ordinary skill in the art will recognize that alternatively, two flowmeters could be used to separately measure carrier gas flow before acquiring vapor and bypass gas 412 through use of two flowmeters placed immediately after junction 224 (dual flowmeters not shown).

Bypass gas 412 passes out one-way valve 230 through control valve 236 to rejoin the carrier gas 418 at junction 242. From junction 242 the flow is directed out one-way valve 248 and control valve 254 to the breathing circuit.

The carrier gas 418 travels from junction 224 through one-way valve 266, control valve 272, control valve 304 and through one-way valve 278 as control valve 308 is closed during normal operation and is only open for purge cycles. At junction 286, the carrier gas picks up vapor from cartridge 400 and proceeds through one-way valve 290 to junction 242 to mix with the bypass gas 412 and move towards the breathing circuit 424 as described above.

Vapor is provided to the system by the application of heat to the cartridge 400 from heat source 460. The control system may receive feedback on the heating process through temperature sensing device 606. As shown in FIG. 3, temperature sensor 606 may be placed between the open cartridge valve 518 and the vaporizer valves 284 and 282 as the process aims to control the temperature of the vapor. Based on the temperature of the vapor, the amount of power provided to the heat source may be regulated. As is known in the art, heat sources may be regulated by increasing the continuous power flow through the heat source or by changing a duty cycle of a constant source so that power is provided to the heat source a greater percentage of the time to increase the temperature of the vapor.

The vapor leaves the reservoir of liquid anesthetic material and passes through cartridge one-way valve 512 and cartridge valve 518. Cartridge valve 518 is actuated by the mechanical insertion of the cartridge 400 into position to become the primary cartridge. This cartridge valve 518 may be of any suitable type such as a Schrader valve.

Once the cartridge valve 518 is open, then vapor may flow through one-way valve 284 and control valve 282 to reach junction 286. When control valve 282 is closed, pressure sensor 618 senses the pressure associated with the vaporizing of the liquid anesthetic material 506. The pressure measurement at pressure sensor 618 may be a gage pressure rather than an absolute pressure as the ambient air pressure measured at pressure sensor 612 allows for the calculation of absolute pressure. The pressure sensed by pressure sensor 618 may be used by the control system to determine if more heat is needed or if the cartridge 400 is exhausted. An exhausted cartridge will not have the predicted pressure for a given temperature.

PURGE CYCLE

As described above, during the operation of the vaporizer, it may be desirable to have a purge cycle. A purge cycle may be desired after the removal of a cartridge without the insertion of a new cartridge within a prescribed time period or before use of a cartridge having a different liquid anesthetic material from the immediate prior cartridge.

During a purge cycle, one-way valve 284 prevents vapor laden gas from leaving the vaporizer into the ambient air.

Bypass gas 412 continues to flow slowly through control valve 236 to avoid condensation of liquid anesthetic material in the vicinity of control valve 236.

During a purge cycle, control valve 304 is closed and control valve 308 is open so that fresh gas is routed to a junction between one-way valve 284 and control valve 282. Purge gas is routed through the passages that would normally contain vapor from the cartridge 400. The fresh gas being used as purge gas cannot go through one-way valve 278 or through closed control valve 304 and thus goes through one-way valve 290 to junction 242. From junction 242 the combination of bypass gas 412 and purge gas goes through one-way valve 318 and control valve 324 to the purge gas system 430 at the facility for handling purge gas and other anesthetic laden gases.

VENTING OF COOLING CARTRIDGE

As the cartridge 400 cools after providing vapor to the vaporizer system, a portion of the liquid anesthetic material returns to liquid from the vapor state. As the vapor returns back to liquid, the pressure within the cartridge 400 would drop as cartridge valve 518 is closed if the cartridge has been removed. Even if that cartridge valve is open as the cartridge is still inserted into the vaporizer, there is not a flow path for gas to go back into the cartridge 400 as such movement is prevented by one-way valve 512, one-way valve 284 and control valve 282.

A pressure relief system may be provided in order to maintain the cartridge interior at near-ambient air pressure 436. When the differential between ambient air pressure 436 and the interior of the cartridge 400 exceeds a preset limit, cartridge pressure relief valve 524 opens to allow ambient air to flow into the cartridge interior through one-way valve 530 and pressure relief valve 524.

VAPORIZER CONTROL SYSTEM

The control system to implement the present invention could be implemented in a variety of ways known to those of skill in the art. The example given below is illustrative of concepts and meant to convey a richer explanation of the present invention rather than implying that the particular illustration is the only way to implement these aspects of the present invention.

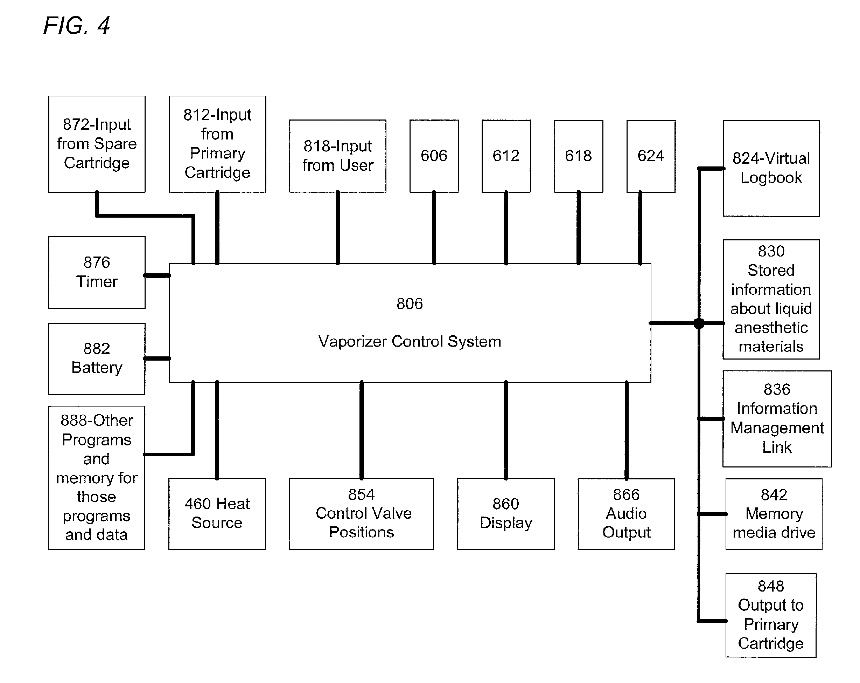

The vaporizer control system 806 works to provide a sufficient amount of vapor to satisfy the requested level of anesthetic vapor requested by the end user (shown in FIG. 4 as Input from User 818). The end user communicates with the vaporizer control system via various user interfaces including one or more large rotary decoders that allow for the rotation of a knob or dial to input the rate of vaporized liquid anesthetic material and other input devices. The other input devices could include touch screen input, keypads or keyboards, a mouse, and even voice recognition software to process input from a microphone.

The communication with the end user must be two-way. The communication from the control system to the end user may be primarily one or more visual displays. An example of a potentially suitable display is a liquid crystal display. The end user may have some ability to navigate through various options to select certain clusters of information for display. The vaporizer control system may have the ability to interrupt the current process of displaying requested information to provide a visual display relevant to the provision of alarm or other warning information to the end user. The alarms and other status information may also be communicated through one or more colored status lights.

Alarms and other information may be provided to the end user, at least in part, by one or more speakers or other audio devices to provide spoken material or to provide sounds indicative of an alarm or other milestones such as a bell sound to indicate that the vaporizer is ready.

As noted above, the vaporizer control system 806 may receive input 812 from the cartridge. The input may be in the form of digital data. This information provided from the cartridge to the vaporizer control system 806 may come from a memory device on the cartridge through a wired or wireless communication path. The communication path may use RFID technology.

The input 812 from the cartridge may include the identity of the specific liquid anesthetic material present in the cartridge placed in the vaporizer to be the primary cartridge. Additional information such as when the cartridge was created or when the cartridge will expire may be conveyed. The cartridge may contain information about the source of the cartridge, possibly a serial number or lot number for the cartridge. The cartridge may convey how much liquid anesthetic material was loaded into the cartridge at the factory or is calculated to remain in the cartridge if operated in a system that writes the estimated volume back into a cartridge after use of the cartridge.

Note, the use of memory within the cartridge to store and communicate the type of liquid anesthetic material contained within that cartridge does not preclude the use of color coding on the outside of the cartridge to communicate the type of liquid anesthetic material contained within.

Optionally, some systems may be implemented to write back to the memory on the cartridge. This is represented on this figure by output 848 for output to the cartridge memory. The output may include the estimated amount of liquid anesthetic material in the cartridge, the date of use of the cartridge, and other information that may be useful in assessing whether to use the cartridge at a future date or in tracing cartridges that have been used in a specific procedure or vaporizer device. Depending on the type of wireless communication system that is used, it may be possible to write to the memory of a recently removed cartridge as long as the cartridge remains within the limited range of the wireless communication system.

The identification of the type of liquid anesthetic material as part of the input 812 from the cartridge allows the vaporizer control system 806 to access stored information about the liquid anesthetic material 830 in order to know the target temperature in order to provide adequate vapor and the pressure that should be observed when the vapor production is adequate.

The vaporizer control system produces the vapor required for the known type of liquid anesthetic material through control of the heat source 460 and the operation of the various control valves via set of control valve positions 854 communicated to the control valves. Some control valves will tend to be operated to be either fully open or fully shut. Other control valves may be operated to have a partially open position such as control valves 272 and 236 which determine the ratio of bypass gas 412 and carrier gas 418.

The vaporizer control system 806 may receive input from one or more sensors such as: vapor pressure sensor 618, ambient air pressure sensor 612, temperature sensor 606 monitoring the temperature of the cartridge vapor, and flowmeter 624 measuring the total flow of fresh gas 406. Control of the various control valves will allow control over both the ratio of bypass gas 412 to carrier gas 418 but also the total amount of fresh gas entering the vaporizer for a given pressure setting of pressure regulator 206.

As noted above, a virtual logbook 824 may be maintained with information about the vaporizer operation during this specific procedure. The vaporizer logbook may include information about the interaction with the end user in addition to the vapor production. The interactions with the end user communicated to the virtual logbook may include the requests made by the end user including a time stamp and any alarms or other status changes communicated to the end user including a time stamp.

Information from the virtual logbook 824 may be communicated to removable memory media via a memory media drive 842. One suitable removable memory media is a USB flash drive. After writing the relevant information for a completed procedure from the virtual logbook 824 to the removable media via memory media drive 842, the information may be immediately deleted from the vaporizer control system's virtual logbook 824 or it may be stored within the vaporizer for some period of time to serve as a back-up in case there is a subsequent need for the data (such as in the instance of the removable memory device being misplaced or corrupted in some way).

Information from the virtual logbook 824 may also be communication to an information management system located outside of the vaporizer via an information management communication link 836. The information management communication link may be implemented via wired or wireless connections.

INPUT FROM SPARE CARTRIDGE

As noted above, the vaporizer may be configured to receive input from the spare cartridge 872 so that the vaporizer control system 806 may know in advance certain relevant attributes of the cartridge that will be used when the primary cartridge becomes exhausted or nearly exhausted. The information to be read from the spare cartridge 872 may include the type of liquid anesthetic material so that inconsistencies between the liquid anesthetic materials in the spare cartridge versus the current primary cartridge may be identified well in advance of the need to use the spare cartridge in case this inconsistency was not intended. The information read from the spare cartridge may include information sufficient to assess if the spare cartridge is expired. The information read from the spare cartridge may include an amount of liquid anesthetic material present in the cartridge. Such information would be useful if the system is implemented to use cartridges with different amounts of liquid anesthetic material within a given liquid anesthetic material type. In such a system, knowing the liquid anesthetic material alone would not be sufficient to know the amount of liquid anesthetic material in the cartridge.

SAFETY IS ENHANCED

By avoiding the process of having an end user refill a cartridge, the risk that an overfilled cartridge may introduce liquid anesthetic material into the interior of the vaporizer during this refilling process is eliminated.

DETECTION OF REMOVAL OF A CARTRIDGE

The removal of a primary cartridge could be detected using one of many conventional methods known to those of skill in the art. One way to detect the removal of a cartridge is when the pressure measured at vapor pressure sensor 618 equals the ambient air pressure 436 as measured at pressure measurement device 612 as there is no longer a primary cartridge to provide vapor pressure.

PURGE CYCLE

During the operation of a purge cycle, the vaporizer control system 806 will set the control valve positions 854 into the purge configuration. The vaporizer control system 806 may use a timer 876 to ensure that the purge configuration of valves is maintained for a prescribed period.

In order to minimize or eliminate opportunities for condensation of vapor within the vaporizer, it may be desirable to start the purge cycle quickly.

The vaporizer control system 806 may be connected to one or more batteries 882 so that a temporary interruption of the primary power supply (not shown) will not impinge on the operation of the vaporizer control system 806 or cause the loss of data stored in volatile memory.

As represented conceptually in block 888, the vaporizer control system 806 may have access to other programs and memories to achieve other functions.

EXAMPLE OF CARTRIDGES AND VAPORIZER

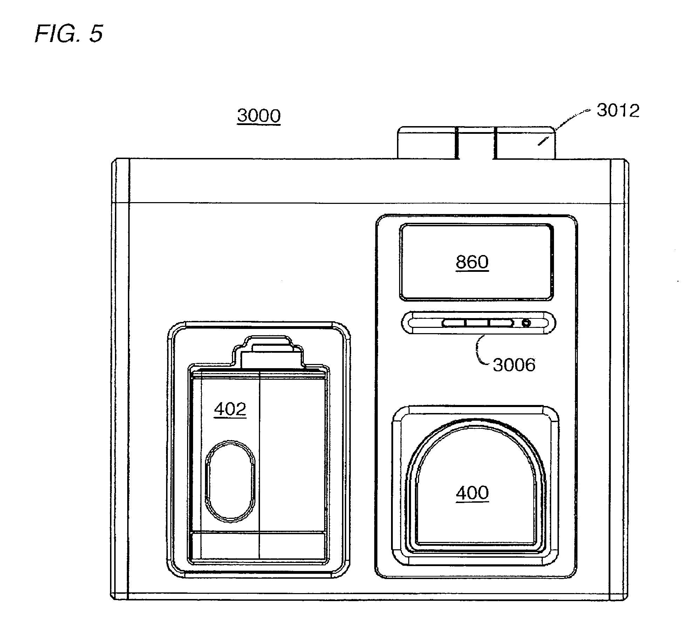

FIG. 5 is a front view of a vaporizer 3000 with both a primary cartridge 400 and a spare cartridge 402. A display 860 is adjacent user input controls 3006 and the front side of rotary input 3012.

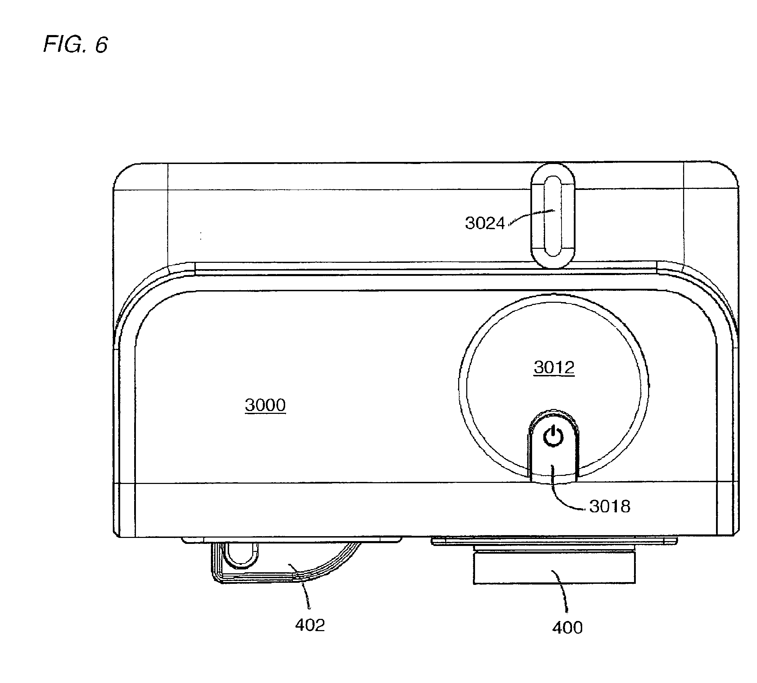

FIG. 6 is a top view of a vaporizer 3000 with both a primary cartridge 400 and a spare cartridge 402. Rotary input 3012 may be used to convey user input to the vaporizer control system such as a request for a change in the rate of delivery of anesthetic vapor.

The rotary decoder 3012 may have a lock 3018 to lock the position of the rotary decoder 3012 so that any subsequent changes in rotary position are made intentionally and not through incidental contact. FIG. 6 provides a view of lock 3024 for interlock fitting

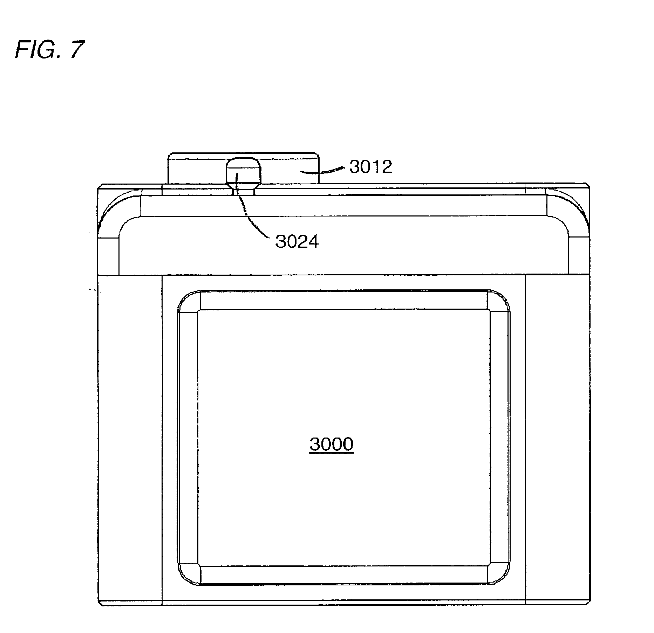

FIG. 7 is a rear view of a vaporizer 3000. Rotary input 3012 and lock 3024 are visible from this view.

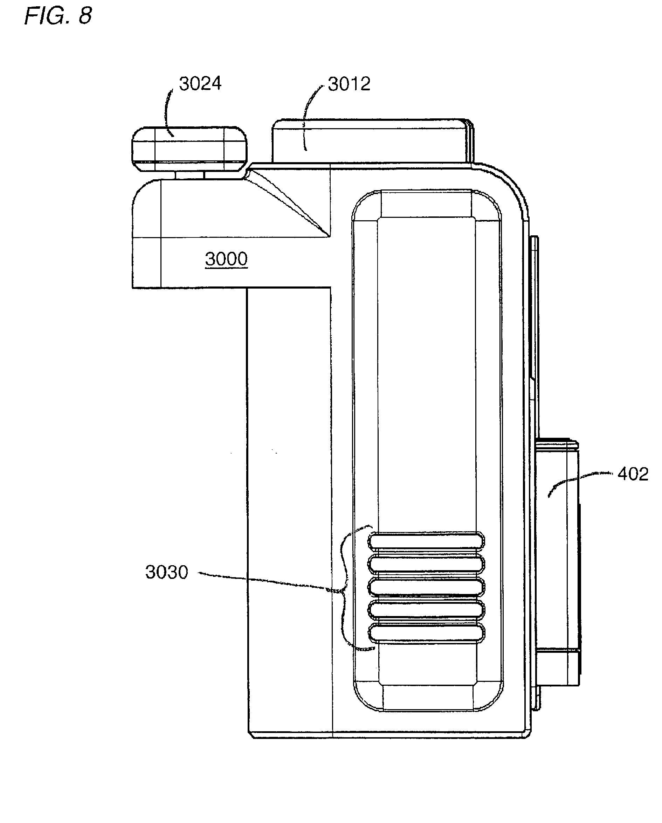

FIG. 8 is a left side view of a vaporizer 3000 with both a primary cartridge (not visible here) and a spare cartridge 402. Rotary input 3012 and lock 3024 are visible from this view. Ventilation to the interior of the vaporizer 3000 may be provided at least in part by vent ports 3030.

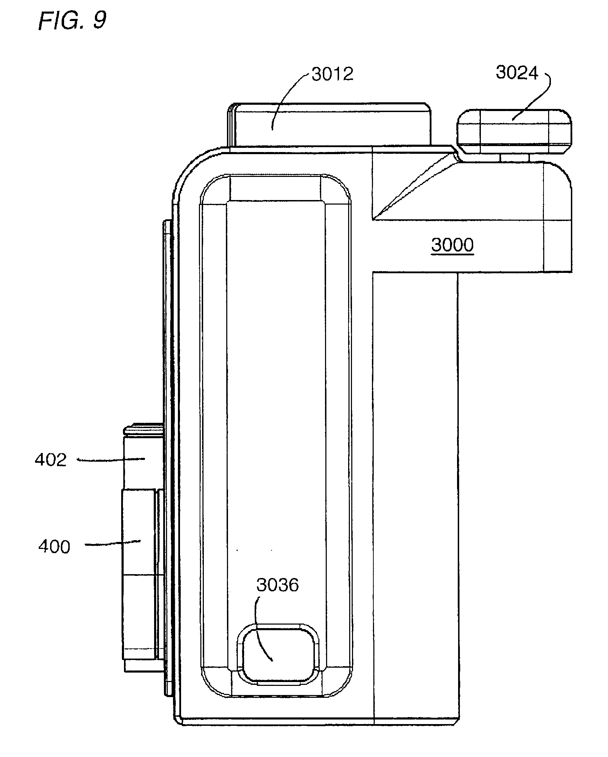

FIG. 9 is a right side view of a vaporizer 3000 with both a primary cartridge 400 and a spare cartridge 402. Rotary input 3012 and lock 3024 are visible from this view. Electrical connections to an external power source can be made through an external power port. The details on what the connectors would look like may vary from country to country but a location for the power port is represented here as port location 3036.

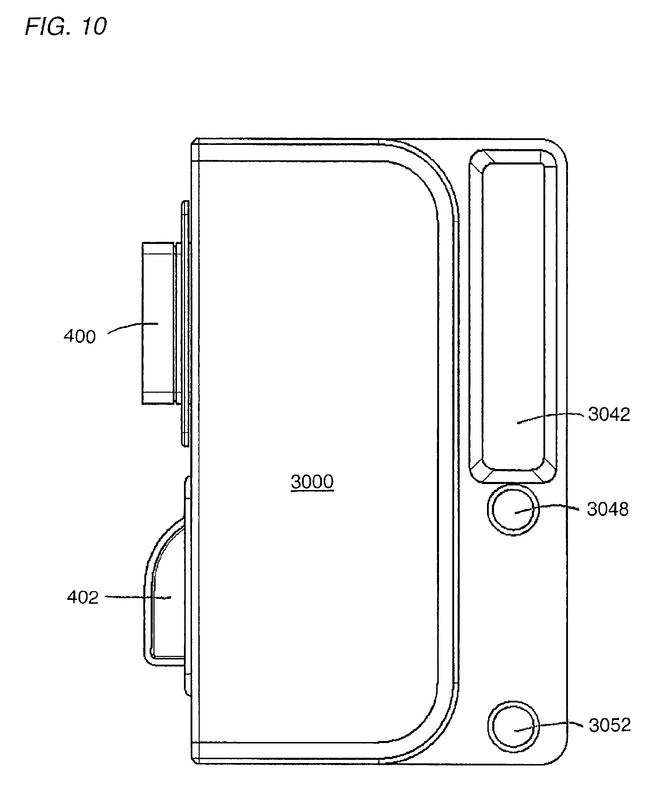

FIG. 10 is a bottom view of a vaporizer 3000 with both a primary cartridge 400 and a spare cartridge 402. Also visible from this view are connection ports 3048 and 3052 to connect the inlet and outlet gas of the vaporizer to an interlock system. Cavity 3042 is shown here to illustrate how a vaporizer could be implemented that is double the width of other prior vaporizers so that a vaporizer might occupy two vaporizer slots on an interlock bar.

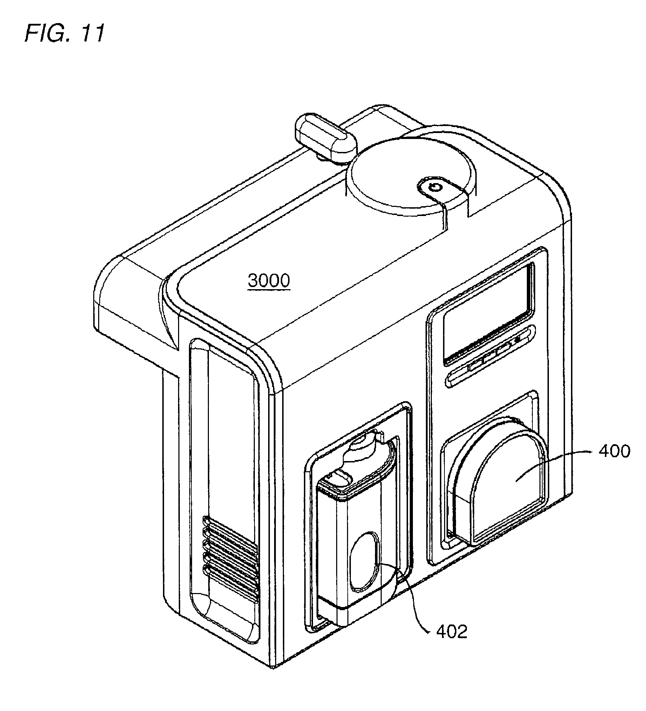

FIG. 11 is a perspective view of the top, left, and front sides of a vaporizer 3000 with both a primary cartridge 400and a spare cartridge 402.

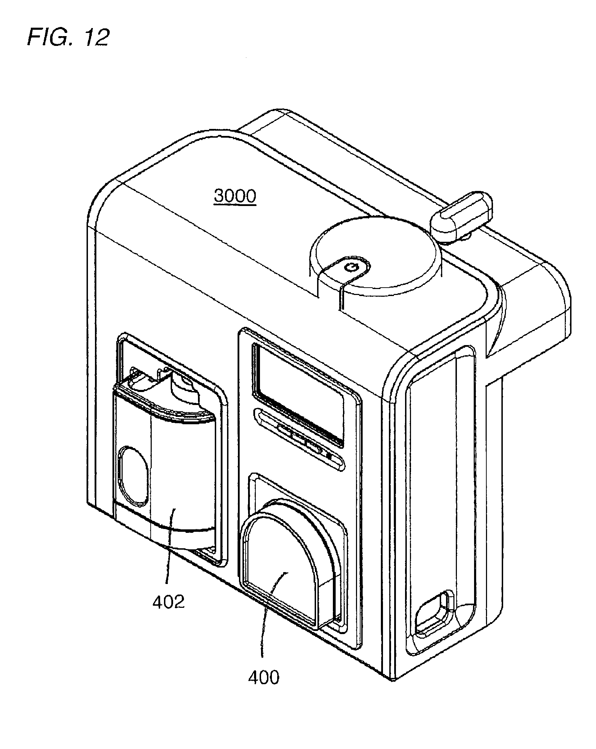

FIG. 12 is a perspective view of the top, right, and front sides of a vaporizer 3000 with both a primary cartridge 400 and a spare cartridge 402.

DETAILS, OPTIONS, AND VARIATIONS

Cartridges could be created with different amounts of liquid anesthetic material so that one cartridge has the appropriate amount of material for a one hour procedure and a second cartridge may have the same material but have enough for a two hour use. A third cartridge may be created with the same material but a four hour supply of that material.

Alternatively, it may be more convenient to use a common volume of liquid anesthetic material such as enough for a two hour procedure and simply use more than one cartridge rather than having a hospital or clinic store many different cartridges with different amounts of liquid anesthetic material. Of course there may be a standard reduced size for use in pediatric surgeries as opposed to cartridges used for adults (adult humans).

If the vaporizer has sufficient capacity to quickly heat the cartridge to the normal operating temperature, then the vaporizer may be used as the sole vaporizer in an anesthesia system. The temporary interruption of the supply of vapor to the breathing circuit will not interrupt the anesthetic effect as the breathing circuit will continue to have decreasing amounts of anesthetic vapor and the patient will have a certain amount of anesthesia already present in the patient and that anesthesia continues to have effect until the anesthesia is removed by the liver which does not happen immediately.

If the vaporizer does not have sufficient capacity to quickly heat the cartridge to the normal operating temperature or if there is a preference for redundant equipment, the vaporizer may be used as one of a set of two or more vaporizers in a system configured to limit the number of vaporizers providing vapor at any one time to a single vaporizer. Using sets of two or more vaporizers with interlocks is well known in the art and need not be addressed here.

HEATING UNIT WITHIN CARTRIDGE

An alternative to having a heating unit within the vaporizer in thermal communication with the primary cartridge is to have a heating unit present in each cartridge. As an example, a resistive heating unit within the cartridge could surround the reservoir of liquid anesthetic material and yet be covered by an insulative layer so that users handling an exhausted cartridge do not have exposure to heated areas of the cartridge. The docking of a cartridge to become a primary cartridge may be configured to establish contact for an electric circuit to provide current to the resistive heating.

One of skill in the art will recognize that other heating systems could be employed within the cartridge. As the cartridge is disposable, a design objective would be to select a heating mechanism that could be implemented inexpensively but yet provide heat in a way that is both quick in heating the cartridge and predictable in how heat is provided with a given amount of energy provided by the vaporizer.

Specific examples have been used within this document in order to augment the description of ways of making and using the disclosed vaporizer and cartridges. Unless explicitly indicated to the contrary, a list of one or more examples is intended to illustrate a point and not provide an exhaustive list of the universe of possibilities.

One of skill in the art will recognize that some of the alternative implementations set forth above are not universally mutually exclusive and that in some cases additional implementations can be created that employ aspects of two or more of the variations described above. Moreover, the scope of the claims which follow covers the range of variations, modifications, and substitutes for the components described herein as would be known to those of skill in the art.

The legal limitations of the scope of the claimed invention are set forth in the claims that follow and extend to cover their legal equivalents. Those unfamiliar with the legal tests for equivalency should consult a person registered to practice before the patent authority which granted this patent such as the United States Patent and Trademark Office or its counterpart.

## Claims

1. A cartridge for use in a vaporizer system for providing anesthetic vapor, the cartridge comprising:
a reservoir at least partially filled with liquid anesthetic material;
a valve in fluid communication with the reservoir, the valve adapted to engage with a structure in a vaporizer system to open the valve to allow for passage of vapor from the reservoir to the vaporizer;
a pressure relief system in fluid communication with the reservoir to prevent pressure in the reservoir from dropping below a prescribed difference from ambient air pressure; and
at least one memory device within the cartridge which may be read by the vaporizer; the memory device containing at least an identification of the liquid anesthetic material contained in the reservoir.

2. The cartridge of claim 1 wherein the cartridge has a path for conducting heat from a vaporizer unit to the reservoir to promote vaporization of the liquid anesthetic material.

3. The cartridge of claim 1 wherein the cartridge has a path for providing current flow from a vaporizer through a heating unit within the cartridge to heat the reservoir using power provided from the vaporizer.

4. The cartridge of claim 1 wherein at least one memory device may be read by the vaporizer using wireless technology.

5. The cartridge of claim 4 wherein at least one memory device may be read by the vaporizer using RFID technology.

6. The cartridge of claim 1 wherein the cartridge contains an electrical communication path that engages with at least one contact on the vaporizer to provide a data path for communication with the at least one memory.

7. The cartridge of claim 1 wherein at least one memory device within the cartridge may receive information from a vaporizer during one interaction with a vaporizer and that information may be accessed later by a vaporizer.

8. The cartridge of claim 1 wherein at least one memory device contains information sufficient for the vaporizer to determine whether the cartridge is beyond an expiration date for use.

9. The cartridge of claim 8 wherein the information is the expiration date for the cartridge.

10. The cartridge of claim 8 wherein the information includes the creation date for the cartridge and does not include the expiration date.

11. The cartridge of claim 1 wherein the at least one memory contains a value for an amount of liquid anesthetic material in the reservoir.

12. The cartridge of claim 11 wherein the amount of liquid anesthetic material is an amount placed into the cartridge at a factory that created the cartridge.

13. The cartridge of claim 12 wherein the cartridge is one of a set of at least two cartridges, each cartridge with the same specific liquid anesthetic material but each cartridge having a different amount of that liquid anesthetic material placed in the cartridge so that one cartridge may be used to provide vapor at a vapor production rate for effective anesthesia for an adult human for an hour longer than another cartridge in the set if used at that same vapor production rate.

14. The cartridge of claim 1 wherein the at least one memory contains a value for the estimated amount of liquid anesthetic material remaining in the cartridge after a previous use in a vaporizer.

15. The cartridge of claim 1 wherein the valve in fluid communication with the reservoir is closed when the cartridge is removed from a vaporizer to prevent vapor from leaving the cartridge to go to the ambient air.

16. The cartridge of claim 1 wherein the cartridge is one of a set of at least two cartridges, each cartridge in the set having an external shape that is the same so that shape cannot be used to distinguish one cartridge from another, but each of the at least two cartridges has a different liquid anesthetic material within the cartridge and each cartridge has at least one memory device containing at least an identification of what liquid anesthetic material is within that cartridge.

17. The cartridge of claim 16 wherein the set of at least two cartridges uses a color code system to indicate the identification of what liquid anesthetic material is within that cartridge so that an end user can use color to select a cartridge having containing a particular liquid anesthetic material.

18. The cartridge of claim 1 wherein the cartridge lacks any means intended for use by an end user to refill a cartridge such that the cartridge is filled only once during a manufacturing process and then not refilled by an end user.

19. A cartridge for use in a vaporizer system for providing anesthetic vapor, the cartridge comprising:
a reservoir for use in holding liquid anesthetic material;
a valve in fluid communication with the reservoir, the valve adapted to engage with a structure in a vaporizer system to open the valve to allow for the passage of vapor from the reservoir to the vaporizer;
a pressure relief system in fluid communication with the reservoir to prevent pressure in the reservoir from dropping below a prescribed difference from ambient air pressure; and
at least one memory device within the cartridge for holding digital data.

20. A vaporizer for use in the production of vapor for use in anesthesia, the vaporizer comprising:
a means for opening a valve on a vaporizer cartridge to establish a path between the vaporizer and the vaporizer cartridge for vapor to flow from the vaporizer cartridge to the vaporizer;
a means for heating a vaporizer cartridge reservoir; and
a means for accessing digital information stored within the vaporizer cartridge to discern at least a type of liquid anesthetic material stored within the vaporizer cartridge.

21. The vaporizer of claim 20 further comprising a means for communicating information about the vaporizer's use of a vaporizer cartridge for storage within the vaporizer cartridge for access by a vaporizer during subsequent use of the vaporizer cartridge.

22. The vaporizer of claim 20 further comprising equipment to read at least one memory within a vaporizer cartridge through a wireless communication link.

23. The vaporizer of claim 22 wherein the equipment uses RFID for the wireless communication link.

24. The vaporizer of claim 20 further comprising contact points to allow digital information to flow from the vaporizer cartridge to the vaporizer without use of wireless communication.

25. The vaporizer of claim 20 further comprising a heating device within the vaporizer and regulated to provide a certain pressure measurement in piping in fluid communication with the interior of the vaporizer cartridge.

26. The vaporizer of claim 20 further comprising electrical contacts for providing power to a heating device within a vaporizer cartridge.

27. The vaporizer of claim 20 wherein the vaporizer uses information from a vaporizer cartridge that indicates the specific liquid anesthetic material in the vaporizer cartridge and control information specific to that specific anesthetic material stored in memory accessible to the vaporizer to operate to produce vapor laden gas sufficient to anesthetize an adult human.

28. The vaporizer of claim 27 wherein the vaporizer adjusts for the ambient air pressure when operating to produce vapor laden gas sufficient to anesthetize an adult human.

29. The vaporizer of claim 20 wherein the vaporizer is adapted to be used with a first vaporizer cartridge containing a first liquid anesthetic material with a first vapor pressure at a first temperature and subsequently used with a second vaporizer cartridge with a second liquid anesthetic material different from the first liquid anesthetic material with a second vapor pressure, different from the first vapor pressure, at the first temperature such that the vaporizer may be used with more than one type of liquid anesthetic material to produce a controlled amount of vapor for use in anesthesia.

30. The vaporizer of claim 20 further comprising a void in the vaporizer for receiving and holding a vaporizer cartridge as a spare cartridge without opening a valve in the spare cartridge so that the vaporizer may be using a first cartridge for the production of vapor and have a second cartridge within the void for receiving and holding the vaporizer cartridge in order to have a spare cartridge nearby for use when the first cartridge has little or no liquid anesthetic material left.

31. The vaporizer of 30 wherein the vaporizer is adapted to read information stored within the spare cartridge including an identification of a type of liquid anesthetic material contained in the spare cartridge so that the vaporizer can compare the type of liquid anesthetic material in the spare cartridge with the type of liquid anesthetic material in the vaporizer cartridge being used by the vaporizer to produce vapor.

32. The vaporizer of claim 20 wherein the vaporizer notes that a new cartridge inserted to replace a first cartridge after use of the first cartridge has a specific type of liquid anesthetic material in the new cartridge that is not the same type of liquid anesthetic material as in the first cartridge and the vaporizer unit provides a warning to communicate this discrepancy to an end user.

33. The vaporizer of claim 32 wherein the vaporizer seeks confirmation from an end user before creating and using vapor from the new cartridge.

34. The vaporizer of claim 20 wherein:
the vaporizer obtains from a newly inserted vaporizer cartridge an indication of an amount of liquid anesthetic material present in the vaporizer cartridge before the start of this use in this vaporizer; and
the vaporizer calculates an amount of liquid anesthetic material removed from the vaporizer cartridge during use and communicates a warning when an estimated time to cartridge exhaustion drops to a specified level.

35. The vaporizer of claim 20 wherein the vaporizer measures a pressure within piping in fluid communication with the vaporizer cartridge and communicates cartridge exhaustion when the measured pressure drops below a target level selected by the vaporizer system in order to provide a requested delivery rate of vapor of the specific liquid anesthetic material.

36. A method of producing vapor from a liquid anesthetic material, the method comprising:
inserting a cartridge into a vaporizer for vapor production;
opening a cartridge valve to provide at least a one-way route of fluid communication between a reservoir of liquid anesthetic material and at least a portion of the vaporizer;
transferring digital information from the cartridge to the vaporizer to provide an identification of a specific liquid anesthetic material contained within the cartridge;
operating the vaporizer based upon vapor production information in memory accessible to the vaporizer that applies to that specific anesthetic material contained within the inserted cartridge but not to all liquid anesthetic materials;
providing heat to the liquid anesthetic material to promote formation of vapor;
controlling the amount of heat provided to the liquid anesthetic material based upon at least one monitoring instrument; and
selectively opening at least one valve within the vaporizer to allow for vapor from the cartridge to be added to gas to create carrier gas.

37. The method of claim 36 further comprising inserting a second cartridge into the vaporizer to serve as a spare cartridge, thus making the other cartridge the primary cartridge so that the spare cartridge may be inserted into the vaporizer as a next primary cartridge after the primary cartridge is removed.

38. The method of claim 36 wherein the vaporizer seeks confirmation from an end user that the end user wishes to use the specific liquid anesthetic material contained within the cartridge.

39. The method of claim 36 wherein the vaporizer receives from an end user a target level of vapor production and the vaporizer produces this target level of vapor production by controlling the operation of a set of at least one valve and the operation of at least one heat source based upon at least one measurement value.

40. The method of claim 39 wherein the heat source is within the vaporizer.

41. The method of claim 39 wherein the heat source is within the cartridge but the vaporizer controls the amount of power provided to the heat source.

42. The method of claim 36 wherein the vaporizer creates a virtual logbook within a memory accessible to the vaporizer with information about the use of the vaporizer including the identification of a specific liquid anesthetic material contained within the cartridge, at least one selected rate of vapor production and at least one time stamp.

43. The method of claim 36 further including:
removing the cartridge from the vaporizer;
after removing the cartridge from the vaporizer, inserting a second cartridge into the vaporizer for use in production of vapor;
opening a cartridge valve to provide at least a one-way route of fluid communication between a reservoir of liquid anesthetic material within the second cartridge and at least a portion of the vaporizer;
transferring digital information from the second cartridge to the vaporizer to provide an identification of a specific liquid anesthetic material contained within the second cartridge; and
checking to ensure that the identification of the specific liquid agent in the second cartridge is the same as the specific liquid agent in the cartridge or receiving confirmation from the end user of intent to switch types of liquid anesthetic material before providing heat to the liquid anesthetic material in the second cartridge to promote formation of vapor.
